# NEW EUROPEAN PATENT SPECIFICATION

(11) **EP 2 528 536 B2**
(45) Date of publication and mention of the opposition decision: **24.03.2021**
(45) Mention of the grant of the patent: 11.11.2015
(21) Application number: 11706973.2
(22) Date of filing: 28.01.2011
(51) Int. Cl.: A61C 19/06, A61K 9/00, A61K 9/70

(54) **DENTAL STRIP FOR ADMINISTRATION OF ORAL TREATMENT**
DENTALER STREIFEN ZUR VERABREICHUNG EINER ORALEN BEHANDLUNG
BANDE DENTAIRE POUR ADMINISTRER UN TRAITEMENT BUCCAL

(30) Priority: 29.01.2010 US 299719 P
(43) Date of publication of application: 05.12.2012
(73) Proprietor: Colgate-Palmolive Company, New York, NY 10022 (US)
(72) Inventor: XU, Guofeng, Plainsboro, New Jersey 08536 (US); VISCIO, David B., Prescott Valley, Arizona 86315-7809 (US); GAFFAR, Abdul, Princeton, New Jersey 08540 (US)
(74) Representative: Wibbelmann, Jobst
(86) International application number: PCT/US2011/023023
(87) International publication number: WO 2011/094613

(56) References cited:
- EP-A1- 1 391 212
- WO-A2-98/55079
- WO-A2-2006/058017
- WO-A2-2007/076396
- US-A- 6 153 222
- US-A- 6 153 222
- US-A1- 2006 292 520
- US-A1- 2006 292 520
- US-B1- 6 343 932
- US-B1- 6 419 906
- Experimental report of Dr. Thomas Inglin dated 9 November 2017

## Description

### BACKGROUND

Sheet-like substrates for administration of oral treatments have been known for some time. A variety of advantages are achieved with such substrates, such as ease of handling, storage and packaging. One disadvantage of such substrates is that the application of the oral treatment is generally limited to the tooth surface, and does not infiltrate the areas between the teeth.

EP-A-1391212, upon which the two-part form of claim 1 is based, discloses oral preparations and supports for oral preparations.

### SUMMARY

The present invention provides a dental strip according to claim 1 that comprises a film backing, an adhesive layer on one surface of the film backing, wherein the film backing is detachable from the adhesive layer, and is characterized in that the adhesive layer including a water-swellable polymer and at least one active agent, wherein the active agent comprises a plaque reducing agent, and wherein the water-swellable polymer further includes an effervescent substance that effervesces on contact with water or saliva. Preferred features are defined in the dependent claims.

Also described herein is a method of delivering an active agent to spaces between teeth in an oral cavity that includes applying a dental strip to teeth in which the dental strip includes a film backing, and an adhesive layer on one surface of the film backing, the adhesive layer including a water-swellable polymer and at least one active agent, wherein the film backing is detachable from the adhesive layer, solubilizing the adhesive layer by saliva present in the oral cavity, wherein the adhesive layer expands and infiltrates the spaces between the teeth thereby delivering the active agent.

Also described herein is a method of removing a substance from spaces between the teeth that includes applying a dental strip to teeth in which the dental strip includes a film backing, and an adhesive layer on one surface of the film backing, the adhesive layer including a water-swellable polymer and at least one active agent, wherein the film backing is detachable from the adhesive layer, solubilizing the adhesive layer by saliva present in the oral cavity, wherein the adhesive layer expands and infiltrates the spaces between the teeth, and removing the dental strip from the teeth.

### BRIEF DESCRIPTION OF THE FIGURES

Fig. 1 shows a dental strip and teeth before application of the dental strip.
Fig. 2 shows teeth with a dental strip applied.
Fig. 3 shows removal of a dental strip from the teeth.

### DETAILED DESCRIPTION

It should be understood that the detailed description and specific examples, while indicating embodiments of the invention, are intended for purposes of illustration only and are not intended to limit the scope of the invention.

The description and specific examples, while indicating embodiments of the invention, are intended for purposes of illustration only and are not intended to limit the scope of the invention. Moreover, recitation of multiple embodiments having stated features is not intended to exclude other embodiments having additional features, or other embodiments incorporating different combinations the stated of features, Specific Examples are provided for illustrative purposes of how to make and use the compositions and methods of this invention and, unless explicitly stated otherwise, are not intended to be a representation that given embodiments of this invention have, or have not, been made or tested.

As used herein, the term "about," when applied to the value for a parameter of a composition or method of this invention, indicates that the calculation or the measurement of the value allows some slight imprecision without having a substantial effect on the chemical or physical attributes of the composition or method. If, for some reason, the imprecision provided by "about" is not otherwise understood in the art with this ordinary meaning, then "about" as used herein indicates a possible variation of up to 5% in the value.

As referred to herein, all compositional percentages are by weight of the total composition, unless otherwise specified.

Some embodiments relate to a removable dental strip for administration of an oral treatment. The dental strip comprises at least two layers, including a film backing and an adhesive layer on one surface of the film backing. The adhesive layer comprises a water-swellable polymer and at least one active agent, wherein the film backing is detachable from the adhesive layer. In some embodiments, the dental strips may be applied to teeth to administer an oral treatment such as delivering an active agent to spaces between the teeth and/or removing substances from areas between teeth.

In some embodiments, the film backing comprises a non-toxic material. Examples of suitable nontoxic substrates include, but are not limited to, textiles, cloth, wood composite, resin, elastomer, and paper. Suitable materials useful as a film backing in the embodiments are discussed in U.S. Patent App. 2002/0187112. Other materials suitable for film backing includes cellulose derivatives such as ethyl cellulose and cellulose acetate, polyvinyl chloride, wax, Parafilms™, polyethylene, polyvinyl alcohol, Teflon™ and their derivatives.

In some embodiments, the film backing includes one or more ethylene oxide polymers. Ethylene oxide polymers useful for a film backing may include homopolymers. Ethylene oxide polymers useful for a film backing may also include mixtures of ethylene oxide polymers of varying molecular weight of 10,000 Daltons to 10,000,000 Daltons. In some embodiments ethylene oxide polymers useful for a film backing include mixtures of ethylene oxide polymers of varying average molecular weight of 100,000 to 1,500,000 Daltons. Such ethylene oxide polymers are commercially available from various sources. Poly(ethylene) oxide polymers having an average molecular weight range of 10,000 to 1,000,000 Daltons are available from the Dow Chemical Company under the tradename "Polyox." Suitable Polyox materials useful in forming the film backing are described in, for example, U.S. Patent Nos. 6,419,906, 6,503,486, 6,514,483.

In some embodiments, a film backing further includes one or more plasticizers. Examples of plasticizers useful for incorporation in a film backing include, but are not limited to, glycols such as propylene glycol, polyethylene glycol (PEG), polyhydric alcohols such as glycerin and sorbitol and glycerol esters such as glycerol triacetate. In some embodiments, the plasticizer is present at about 1 to about 30% by weight of the film. In other embodiments, the plasticizer is present at about 3 to about 20% by weight.

The ethylene oxide polymer film of the present invention may be prepared using conventional extrusion or solvent casting processes. For example, to prepare a film by solvent casting poly(ethylene) oxide, the ethylene oxide polymer or mixture of polymers may be dissolved in a sufficient amount of a solvent that is compatible with the polymer. In some embodiments, examples of suitable solvents include but are not limited to water, alcohols, acetone, ethyl acetate or mixtures thereof.

In embodiments that optionally include a plasticizer, after a solution of the polymer has been formed, a plasticizer is added with stirring, and heat is applied if necessary to aid dissolution until a clear and homogeneous solution of polymer and plasticizer has been formed. The solution may be coated onto a suitable carrier material and dried to form a film.

In some embodiments, the carrier material has a surface tension that allows the polymer solution to spread evenly across the intended carrier width without soaking in to form a destructive bond between the two substrates. Examples of suitable carrier materials include glass, stainless steel, teflon, polyethylene-impregnated kraft paper, and the like. In some embodiments, the film is dried. In some embodiments, the film is coated with an adhesive layer. In some embodiments the film is cut into pieces of suitable size and shape.

In some embodiments, the film backing has a thickness of 20 to 1500 mm, preferably a thickness of 30 to 1000mm. The film backing has a length of about 2 to about 20 cm, preferably a length of about 4 to about 14 cm. The film backing has a width of about 1 to about 6 cm, preferably a length of about 2 to about 4 cm.

An adhesive layer is applied to one surface of the film backing. In some embodiments, the adhesive layer is coated onto the film backing. In some embodiments the adhesive layer is hydratable. The adhesive layer includes a water-swellable polymer and at least one active agent.

In some embodiments, the adhesive layer may exhibit tackiness, or an adhesive quality, prior to coming in contact with water or saliva. In other embodiments, the adhesive layer exhibits increased tackiness or adhesive quality upon coming in contact with water or saliva. The adhesive layer includes a water-swellable polymer and at least one active agent. In some embodiments, the adhesive layer includes suitable additives such as flavorants, colorants, and/or preservatives.

In some embodiments, the adhesive layer includes or has a substantial portion of swelling agents. Suitable swelling agents include, for example, those known as absorbent polymers in sanitary products, such as diapers, panty liners, in feminine hygiene, and the like, and are known as superabsorbent polymers. Such swellable substances absorb aqueous liquids and spontaneously combine with them to form a relatively stable gel. They are capable of absorbing and permanently retaining many times their weight of aqueous liquids. The resultant gel has chain-like molecules linked into a three-dimensional network and embedded in a liquid medium.

Examples of water-swellable polymer useful for an adhesive layer are described in U.S. Patent 6,153,222. In some embodiments, the water-swellable polymer includes a hydrogel. In some embodiments, the hydrogel includes cross-linked carboxyvinyl copolymers and/or cross-linked polyvinyl pyrrolidones. Other materials suitable for the adhesive layers include polyethylene oxide (also known as Polyox), carboxymethyl cellulose, hydroxypropyl cellulose, hydroxypropyl methyl cellulose, polyacrylic acid and its derivatives, Carbopol, polyvinyl alcohol and alginate. The weight of the adhesive materials can range from about 1mg to 20 gm, preferably from about 20 mg to 5 gm.

In some embodiments, the water-swellable polymer expands on contact with water or saliva and assumes several times its original volume. The polymer includes substances that are effervescent on contact with water or saliva. Such substances may be sodium bicarbonate, and citric or ascorbic acid, or other physiologically acceptable acids. If these foams are used, rapid disintegration of the adhesive layer, which is desirable in some cases, may selectively be further accelerated by the developing increase in volume of the water-swellable polymer.

In some embodiments, the adhesive layer and/or water-swellable polymer that may be incorporated into the adhesive layer is impregnated with one or more active agents. In other embodiments, the adhesive layer and/or water-swellable polymer is coated with one or more active agents.

The adhesive layer includes one or more active agents that prevent, treat and/or reduce the symptoms related to various oral or systemic diseases or conditions.

The active agent comprises a plaque-reducing agent, such as enzymes and non-enzyme proteins. Examples of plaque-reducing enzymes include papain, krillase, and glucoamylase. Examples of plaque-reducing non-enzyme proteins include milk protein. In some embodiments, the active agent includes silica.

The film backing is detachable from the adhesive layer. In some embodiments, the film backing may be detachable from the adhesive layer before, during, or after the administration of an oral treatment. In some embodiments, the film backing is detachable from the adhesive layer upon application of pressure to the film backing. For example, pressure may be applied to the film backing by one's fingers when the dental strip is applied to the teeth.

In some embodiments, the dental strip may be applied to the teeth for administration of an oral treatment. In some embodiments, the oral treatment involves delivery of an active agent to the teeth and to the spaces between the teeth. In some embodiments, the active agent inhibits bacterial growth in the interdental space. In some embodiments, the oral treatment involves removal of substances from the teeth and the spaces between the teeth.

The dental strips may be applied to the teeth for administration of an oral treatment. In some embodiments, the oral treatment involves delivery of an active agent to the teeth and to the spaces between the teeth. In some embodiments, the oral treatment involves removal of substances from the teeth and the spaces between the teeth.

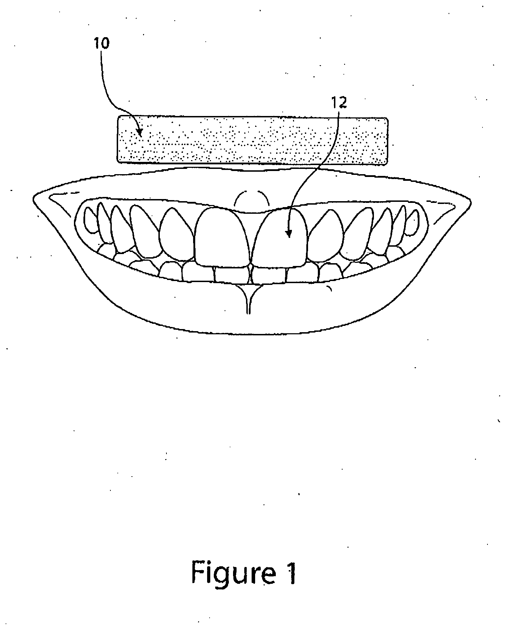

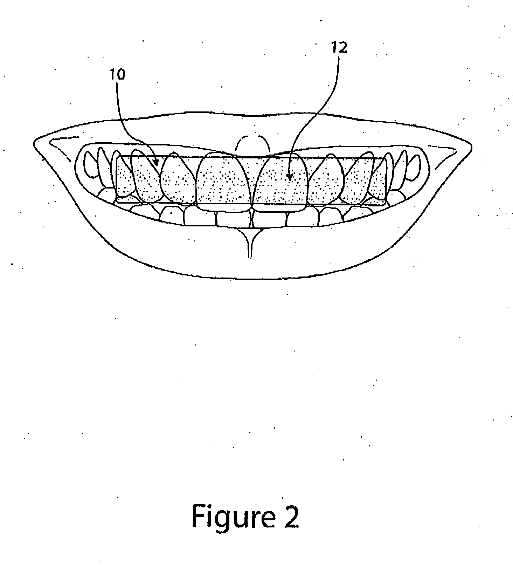

Figure 1 shows dental strip 10 and teeth 12 before application of dental strip 10 to teeth 12. Dental strip 10 may be applied to teeth 12 with the adhesive layer between teeth 12 and the film backing, as shown in Figure 2. In some embodiments, pressure may be applied to push dental strip 10 against teeth 12. In some embodiments, pressure applied to dental strip 10 may cause the adhesive layer to infiltrate the spaces between teeth 12. In some embodiments, such pressure may detach the film backing from all or part of the adhesive layer.

Saliva present in the oral cavity solubilizes the adhesive layer. Upon contact with the saliva, the adhesive layer, which includes a water-swellable polymer, expands. The adhesive layer expands and infiltrates the spaces between teeth 12. Active agents are delivered to the spaces between teeth 12 from the adhesive layer. In some embodiments, the film backing detaches from all or part of the adhesive layer upon expansion of the adhesive layer.

In some embodiments, the active agent, which comprises a plaque-reducing agent, removes substances from teeth 12 and the spaces between teeth 12. Such substances may include, for example, plaque, bacteria, or food particles. In some embodiments, the active agent dissolves the substance. In some embodiments, the active agent loosens the substance. In some embodiments, the active agent attaches to the substance and reduces its adherence to the teeth and surrounding tissue. In this embodiment, the substance (and active agent attached thereto), can then be removed by, for example, rinsing or brushing.

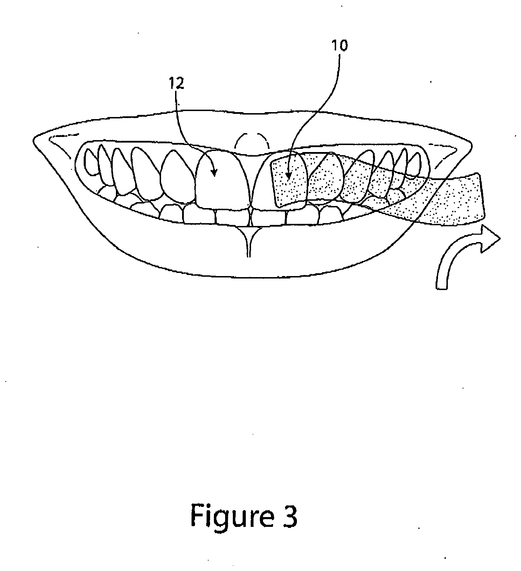

Dental strip 10 may be left on teeth 12 for an amount of time necessary to achieve the intended results of the oral treatment. As shown in Figure 3, dental strip 10 may then be removed from teeth 12. In some embodiments, the film backing and the adhesive layer remain attached to each other upon removal of dental strip 10. The substances may be removed from teeth 12 and the spaces between teeth 12 upon removal of dental strip 10.

In some embodiments, the film backing may be detached from all or part of the adhesive layer upon attachment of the adhesive layer to teeth 12. In some embodiments, the adhesive layer remains on teeth 12 and in the spaces between teeth 12 after removal of the film backing. The active agent may continue to remove substances from teeth 12 and the spaces between teeth 12 after the film backing is removed. In some embodiments, the adhesive layer and water-swellable polymer(s) and active agent(s) and any substances that may be adhered to the adhesive material of the adhesive layer may be removed from teeth 12 and the spaces between teeth 12 by mechanical abrasion upon removal of the film backing.

## Claims

1. A dental strip (10) comprising:
a film backing, and
an adhesive layer on one surface of the film backing, wherein the film backing is detachable from the adhesive layer, **characterised in that** the adhesive layer comprises a water-swellable polymer and at least one active agent,
wherein the active agent comprises a plaque-reducing agent,
and wherein the water-swellable polymer further includes an effervescent substance that effervesces on contact with water or saliva.

2. The dental strip (10) of claim 1, wherein the plaque-reducing agent comprises an enzyme, optionally wherein the enzyme comprises at least one member selected from the group consisting of papain, krillase, and glucoamylase.

3. The dental strip (10) of any one of claims 1-2, wherein the plaque-reducing agent comprises a non-enzyme protein, optionally wherein the non-enzyme protein comprises milk protein.

4. The dental strip (10) of any one of claims 1-3, wherein the active agent comprises silica.

5. The dental strip (10) of any one of claims 1-4, wherein the active agent comprises at least one member selected from the group consisting of triclosan, chlorhexidine, alexidine, hexetidine, sanguinarine, benzalkonium chloride, salicylanilide, domiphen bromide, cetylpyridinium chloride (CPC), tetradecylpyridinium chloride (TPC), N-tetradecyl-4-ethylpyridinium chloride (TDEPC), octenidine, octapinol, nisin, a zinc ion source, a copper ion source, an essential oil, a furanone, an enzyme, a peptide, a protein, a bacteriocin and salts thereof, and honokiol.

6. The dental strip (10) of any one of claims 1-5, wherein the water-swellable polymer comprises a hydrogel, optionally wherein the hydrogel comprises a cross-linked carboxyvinyl copolymer and/or a cross-linked polyvinyl pyrrolidone.

7. The dental strip (10) of any one of claims 1-6, wherein the film backing comprises a non-toxic substrate.

8. The dental strip (10) of any one of claims 1-7, wherein the film backing comprises at least one member selected from the group consisting of textiles, cloth, wood composite, resin, elastomer, and paper.

9. The dental strip (10) of any one of claims 1-8, wherein the film backing comprises an ethylene oxide polymer, optionally wherein the film backing comprises a mixture of ethylene oxide polymers of varying molecular weight of 10,000 Daltons to 10,000,000 Daltons, optionally 100,000 Daltons to 1,500,000 Daltons.

10. The dental strip (10) of any one of claims 1-9, wherein the film backing comprises homopolymers.

11. The dental strip (10) of any one of claims 1-10, wherein the film backing is detachable from the adhesive layer before an oral treatment.

12. The dental strip (10) of any one of claims 1-10, wherein the film backing is detachable from the adhesive layer during an oral treatment.

13. The dental strip (10) of any one of claims 1-10, wherein the film backing is detachable from the adhesive layer after an oral treatment.

14. The dental strip (10) of any one of claims 1-13, wherein the film backing is detachable from the adhesive layer upon application of pressure to the film backing.

15. The dental strip (10) of any one of claims 1-5, wherein the film backing has a thickness of 20 to 1500 µm.

## Patentansprüche

1. Ein dentaler Streifen (10), der Folgendes beinhaltet:
eine Trägerfolie, und
eine Klebeschicht auf einer Oberfläche der Trägerfolie, wobei die Trägerfolie von der Klebeschicht abnehmbar ist, **dadurch gekennzeichnet, dass** die Klebeschicht ein wasserquellfähiges Polymer und mindestens einen Wirkstoff beinhaltet,
wobei der Wirkstoff ein plaquereduzierendes Mittel beinhaltet
und wobei das wasserquellbare Polymer weiterhin eine aufschäumende Substanz enthält, die bei Kontakt mit Wasser oder Speichel aufschäumt.

2. Der Zahnstreifen (10) gemäß Anspruch 1, wobei das plaquereduzierende Mittel ein Enzym beinhaltet, wobei das Enzym optional mindestens ein Mitglied beinhaltet, das aus der Gruppe ausgewählt ist, die aus Papain, Krillase und Glucoamylase besteht.

3. Der Zahnstreifen (10) gemäß einem der Ansprüche 1-2, wobei das plaquereduzierende Mittel ein Nicht- Enzymprotein beinhaltet, wobei das Nicht-Enzymprotein optional Milchprotein beinhaltet.

4. Der Zahnstreifen (10) gemäß einem der Ansprüche 1-3, wobei der Wirkstoff Siliciumdioxid beinhaltet.

5. Der Zahnstreifen (10) gemäß einem der Ansprüche 1-4, wobei der Wirkstoff mindestens ein Mitglied beinhaltet, das aus der Gruppe ausgewählt ist, die aus Folgenden besteht: Triclosan, Chlorhexidin, Alexidin, Hexetidin, Sanguinarin, Benzalkoniumchlorid, Salicylanilid, Domiphenbromid, Cetylpyridiniumchlorid (CPC), Tetradecylpyridiniumchlorid (TPC), N-Tetradecyl-4-ethylpyridiniumchlorid (TDEPC), Octenidin, Octapinol, Nisin, einer Zinkionenquelle, einer Kupferionenquelle, einem ätherischen Öl, einem Furanon, einem Enzym, einem Peptid, einem Protein, einem Bacteriocin und Salzen davon, und Honokiol.

6. Der Zahnstreifen (10) gemäß einem der Ansprüche 1-5, wobei das wasserquellfähige Polymer ein Hydrogel beinhaltet, wobei das Hydrogel optional ein vernetztes Carboxyvinyl-Copolymer und/oder ein vernetztes Polyvinylpyrrolidon beinhaltet.

7. Der Zahnstreifen (10) gemäß einem der Ansprüche 1-6, wobei die Trägerfolie ein ungiftiges Substrat beinhaltet.

8. Der Zahnstreifen (10) gemäß einem der Ansprüche 1-7, wobei die Trägerfolie mindestens ein Mitglied beinhaltet, das aus der Gruppe ausgewählt ist, die aus Textilien, Tuch, Holzverbundstoff, Harz, Elastomer und Papier besteht.

9. Der Zahnstreifen (10) gemäß einem der Ansprüche 1-8, wobei die Trägerfolie ein Ethylenoxidpolymer beinhaltet, wobei die Trägerfolie optional ein Gemisch von Ethylenoxidpolymeren mit unterschiedlichem Molekulargewicht von 10.000 Dalton bis 10.000.000 Dalton, optional von 100.000 Dalton bis 1.500.000 Dalton beinhaltet.

10. Der Zahnstreifen (10) gemäß einem der Ansprüche 1-9, wobei die Trägerfolie Homopolymere beinhaltet.

11. Der Zahnstreifen (10) gemäß einem der Ansprüche 1-10, wobei die Trägerfolie vor einer oralen Behandlung von der Klebeschicht abnehmbar ist.

12. Der Zahnstreifen (10) gemäß einem der Ansprüche 1-10, wobei die Trägerfolie während einer oralen Behandlung von der Klebeschicht abnehmbar ist.

13. Der Zahnstreifen (10) gemäß einem der Ansprüche 1-10, wobei die Trägerfolie nach einer oralen Behandlung von der Klebeschicht abnehmbar ist.

14. Der Zahnstreifen (10) gemäß einem der Ansprüche 1-13, wobei die Trägerfolie nach Druckanwendung auf die Trägerfolie von der Klebeschicht abnehmbar ist.

15. Der Zahnstreifen (10) gemäß einem der Ansprüche 1-5, wobei die Trägerfolie eine Dicke von 20 bis 1500 mm aufweist.

## Revendications

1. Bande dentaire (10) comprenant:
un support de film, et
une couche adhésive sur une surface du support de film, dans laquelle le support de film est détachable de la couche adhésive, **caractérisée en ce que** la couche adhésive comprend un polymère gonflable à l'eau et au moins un agent actif,
dans laquelle l'agent actif comprend un agent de réduction de plaque,
et dans laquelle le polymère gonflable à l'eau inclut en outre une substance effervescente qui entre en effervescence au contact d'eau ou de salive.

2. Bande dentaire (10) selon la revendication 1, dans laquelle l'agent de réduction de plaque comprend une enzyme, éventuellement dans laquelle l'enzyme comprend au moins un élément choisi parmi le groupe consistant en la papaïne, la krillase et la glucoamylase.

3. Bande dentaire (10) selon l'une quelconque des revendications 1 à 2, dans laquelle l'agent de réduction de plaque comprend une protéine non enzymatique, éventuellement dans laquelle la protéine non enzymatique comprend une protéine du lait.

4. Bande dentaire (10) selon l'une quelconque des revendications 1 à 3, dans laquelle l'agent actif comprend de la silice.

5. Bande dentaire (10) selon l'une quelconque des revendications 1 à 4, dans laquelle l'agent actif comprend au moins un élément choisi parmi le groupe consistant en le triclosan, la chlorhexidine, l'alexidine, l'hexétidine, la sanguinarine, le chlorure de benzalkonium, le salicylanilide, le bromure de domiphène, le chlorure de cétylpyridinium (CPC), le chlorure de tétradécylpyridinium (TPC), le chlorure de N-tétradécyl-4-éthylpyridinium (TDEPC), l'octénidine, l'octapinol, la nisine, une source d'ions zinc, une source d'ions cuivre, une huile essentielle, une furanone, une enzyme, un peptide, une protéine, une bactériocine et des sels de ceux-ci et l'honokiol.

6. Bande dentaire (10) selon l'une quelconque des revendications 1 à 5, dans laquelle le polymère gonflable à l'eau comprend un hydrogel, éventuellement dans laquelle l'hydrogel comprend un copolymère de carboxyvinyle réticulé et/ou une polyvinylpyrrolidone réticulée.

7. Bande dentaire (10) selon l'une quelconque des revendications 1 à 6, dans laquelle le support de film comprend un substrat non toxique.

8. Bande dentaire (10) selon l'une quelconque des revendications 1 à 7, dans laquelle le support de film comprend au moins un élément choisi parmi le groupe consistant en des textiles, un tissu, un composite de bois, une résine, un élastomère et un papier.

9. Bande dentaire (10) selon l'une quelconque des revendications 1 à 8, dans laquelle le support de film comprend un polymère d'oxyde d'éthylène, éventuellement dans laquelle le support de film comprend un mélange de polymères d'oxyde d'éthylène de divers poids moléculaires allant de 10 000 Daltons à 10 000 000 Daltons, éventuellement 100 000 Daltons à 1 500 000 Daltons.

10. Bande dentaire (10) selon l'une quelconque des revendications 1 à 9, dans laquelle le support de film comprend des homopolymères.

11. Bande dentaire (10) selon l'une quelconque des revendications 1 à 10, dans laquelle le support de film est détachable de la couche adhésive avant un traitement buccal.

12. Bande dentaire (10) selon l'une quelconque des revendications 1 à 10, dans laquelle le support de film est détachable de la couche adhésive au cours d'un traitement buccal.

13. Bande dentaire (10) selon l'une quelconque des revendications 1 à 10, dans laquelle le support de film est détachable de la couche adhésive après un traitement buccal.

14. Bande dentaire (10) selon l'une quelconque des revendications 1 à 13, dans laquelle le support de film est détachable de la couche adhésive par l'application d'une pression au support de film.

15. Bande dentaire (10) selon l'une quelconque des revendications 1 à 5, dans laquelle le support de film a une épaisseur de 20 à 1 500 µm.
